# EUROPEAN PATENT APPLICATION

(11) **EP 4 179 981 A1**
(43) Date of publication of application: **17.05.2023**
(21) Application number: 20944761.4
(22) Date of filing: 10.07.2020
(51) Int. Cl.: A61B 8/12

(54) **MEDICAL DEVICE AND IMAGE GENERATION METHOD**

(71) Applicant: Asahi Intecc Co., Ltd., Seto-shi, Aichi 489-0071 (JP)
(72) Inventor: SHIMOGAMI Manabu, Seto-shi, Aichi 489-0071 (JP); OSHIMA Fumiyoshi, Seto-shi, Aichi 489-0071 (JP)
(74) Representative: Winter, Brandl - Partnerschaft mbB
(86) International application number: PCT/JP2020/026954
(87) International publication number: WO 2022/009407

(57) **Abstract**

A medical apparatus comprising: an ultrasonic information acquisition unit that acquires ultrasonic information of inside of a living body obtained from an ultrasonic probe that has an ultrasonic sensor and emits ultrasonic waves inside the living body; a device magnetic field information acquisition unit that acquires device magnetic field information relating to a magnetic field generated from a medical device inserted in a blood vessel inside the living body; an echo image generation unit that generates an ultrasonic echo image of the blood vessel from the ultrasonic information; and a device position image generation unit that generates a device position image indicating the position of the medical device inside the blood vessel, based on the ultrasonic echo image and the device magnetic field information.

## Description

### TECHNICAL FIELD

The disclosed embodiments relate to a medical apparatus and an image generation method.

### BACKGROUND ART

A technique is known that uses ultrasonic waves to obtain, from a human body, information about the inside of the human body. For example,

Patent Literature 1 discloses a vascular endothelial function examination method for acquiring an ultrasonic echo image of a blood vessel (also referred to as "blood vessel ultrasonic image") using an ultrasonic probe, and calculating the diameter of the blood vessel from the acquired image.

On the other hand, a technique is known that uses a magnetic force to obtain the position of a medical device inserted inside a human body. For example, Patent Literature 2 discloses a position detection method for detecting the position of a medical device (also referred to as "medical equipment") by using a magnetic sensor to detect the magnetic force emitted by a magnetic field generation source provided in the medical device.

### CITATION LIST

### Patent Literature

Patent Literature 1: JP 2003-245280 A
Patent Literature 2: JP H10-230016 A

### SUMMARY OF INVENTION

### TECHNICAL PROBLEM

Here, the ultrasonic echo image obtained in the technique described in Patent Literature 1 is a longitudinal cross-section of the blood vessel (long axis cross-section of the blood vessel) that corresponds to the position of the ultrasonic probe. Therefore, when the position of the ultrasonic probe and the medical device inserted inside the human body coincide, the operator is able to confirm the medical device inside the blood vessel in the ultrasonic echo image. However, when a displacement occurs in the position of the ultrasonic probe and the medical device, the operator is unable to confirm the medical device inside the blood vessel in the ultrasonic echo image, causing the problem of effort and time being required for the procedure. Furthermore, in the technique described in Patent Literature 2, there is a problem that the position of the medical device inside the blood vessel cannot be confirmed.

Such problems are generally common across medical devices such as catheters, guide wires, and endoscopes, which are inserted into organs (biological lumens) inside the human body including the blood vascular system, lymphatic system, biliary system, urinary system, respiratory system, digestive system, secretory glands, and reproductive organs.

The disclosed embodiments have been made to solve at least some of the problems described above, and an object of the disclosed embodiments is to provide a medical apparatus that is capable of presenting an operator with the position of the medical device inside a blood vessel.

### SOLUTION TO PROBLEM

The disclosed embodiments have been made to solve at least some of the problems described above, and can be implemented as the following aspects.
(1) According to an aspect of the disclosed embodiments, a medical apparatus is provided. The medical apparatus comprises: an ultrasonic information acquisition unit that acquires in-vivo ultrasonic information of inside of a living body obtained from an ultrasonic probe that has an ultrasonic sensor and emits ultrasonic waves inside the living body; a device magnetic field information acquisition unit that obtains device magnetic field information relating to a magnetic field generated from a medical device inserted in a blood vessel inside the living body; an echo image generation unit that generates an ultrasonic echo image of the blood vessel from the ultrasonic information; and a device position image generation unit that generates a device position image indicating a position of the medical device inside the blood vessel, based on the ultrasonic echo image and the device magnetic field information.
   According to this configuration, the medical apparatus is provided with a device position image generation unit that generates a device position image that indicates the position of the medical device inside the blood vessel from an ultrasonic echo image and device magnetic field information. This allows the operator such as a physician to use the device position image to confirm the position of the medical device inside the blood vessel, which enables the medical device inside the blood vessel to be easily moved to the intended position. As a result, the effort and time required for the procedure can be reduced, and the accuracy of the procedure can be improved.
(2) The medical apparatus of the above aspect may further include the ultrasonic probe provided with a magnetic field generation unit, and a probe magnetic field information acquisition unit that acquires probe magnetic field information relating to a magnetic field generated from the ultrasonic probe, wherein the device position image generation unit generates, with respect to a reference plane that traverses the blood vessel defined by the probe magnetic field information, the device position image that indicates whether the medical device is positioned on one side or positioned on another side of the reference plane.
   According to this configuration, the medical apparatus includes an ultrasonic probe provided with a magnetic field generation unit, and a probe magnetic field information acquisition unit that acquires probe magnetic field information relating to the magnetic field generated from the ultrasonic probe. As a result, the medical apparatus uses the probe magnetic field information to align the longitudinal cross-section of the blood vessel (in other words, a reference plane that traverses the blood vessel) appearing in the ultrasonic echo image and the device magnetic field information, which then enables the position of the medical device inside the blood vessel to be identified from the device magnetic field information image, and the device position image to be generated. Furthermore, the device position image is an image that indicates whether the medical device is positioned, with respect to the longitudinal cross-section of the blood vessel (the reference plane that traverses the blood vessel) appearing in the ultrasonic echo image, on one side or positioned on another side of the longitudinal cross-section. As a result, the operator can check the device position image to easily grasp the direction that the medical device should be advanced.
(3) The medical apparatus of the above aspect may further include the ultrasonic probe provided with a magnetic sensor having a fixed positional relationship with the ultrasonic sensor, wherein the device position image generation unit generates, with respect to a reference plane that traverses the blood vessel, the device position image that indicates whether the medical device is positioned on one side or positioned on another side of the reference plane using the positional relationship between the ultrasonic sensor and the magnetic sensor.
   According to this configuration, the medical apparatus includes an ultrasonic probe provided with a magnetic sensor having a fixed positional relationship with the ultrasonic sensor. That is, according to the present configuration, because the positional relationship between the ultrasonic sensor of the ultrasonic probe and the magnetic sensor is already known, the medical apparatus is capable of identifying the position of the medical device inside the blood vessel from the device magnetic field information, and generating the device position image without aligning the longitudinal cross-section of the blood vessel (the reference plane that traverses the blood vessel) appearing in the ultrasonic echo image and the device magnetic field information. Furthermore, because the distance from the magnetic sensor and the ultrasonic sensor to the blood vessel into which the medical device is inserted can be made shorter, the detection accuracy of the magnetic sensor and the ultrasonic sensor can be improved, and the reliability of the position information of the medical device obtained by the magnetic sensor can be improved. In addition, the device position image is an image that indicates whether the medical device is positioned, with respect to the longitudinal cross-section of the blood vessel appearing in the ultrasonic echo image, on one side or positioned on another side of the longitudinal cross-section. As a result, the operator can check the device position image to easily grasp the direction that the medical device should be advanced.
(4) In the medical apparatus of the above aspect, the device position image generation unit may generate the device position image, in which an image indicating a position of the reference plane that traverses the blood vessel, and an image indicating a position of the medical device are superimposed on an image representing a transverse cross-section of the blood vessel.
   According to this configuration, the device position image generation unit generates the device position image, in which an image indicating the position of the longitudinal cross-section of the blood vessel, and an image indicating the position of the medical device are superimposed on an image representing a transverse cross-section of the blood vessel. As a result, the operator can check the device position image to intuitively grasp the position of the medical device inside the blood vessel, and the direction that the medical device should be advanced.
(5) In the medical apparatus of the above aspect, the device position image generation unit may generate the device position image, in which a plane representing the reference plane that traverses the blood vessel, and a three-dimensional image of a distal end portion of the medical device are superimposed on a three-dimensional image of the blood vessel.
   According to this configuration, the device position image generation unit generates the device position image such that a plane representing the longitudinal cross-section of the blood vessel, and a three-dimensional image of the distal end portion of the medical device are superimposed on a three-dimensional image of the blood vessel. As a result, the operator can check the device position image to even more intuitively grasp the position of the medical device inside the blood vessel, and the direction that the medical device should be advanced.
(6) The medical apparatus of the above aspect may further include a synthetic image generation unit that generates a synthetic image in which the ultrasonic echo image and the device position image are combined.
   According to this configuration, the medical apparatus includes the synthetic image generation unit that generates a synthetic image by combining the ultrasonic echo image and the device position image. As a result, the operator is capable of checking the ultrasonic echo image and the device position image at the same time while performing the procedure, which enables the effort and time required for the procedure to be further reduced, while also further improving the accuracy of the procedure. Furthermore, even when a displacement occurs in the position of the ultrasonic probe and the medical device, and the medical device can no longer be confirmed in the ultrasonic echo image, the operator can check the device position image to easily grasp the direction that the medical device should be advanced, such that the medical device can easily be returned to the intended position (such as on the longitudinal cross-section of the blood vessel that appears in the ultrasonic echo image).

The disclosed embodiments may be realized in various modes, and may be realized in modes such as image generation devices that generate images for display, image generation methods, examination devices, examination methods, medical systems, production methods of these devices and systems, and computer programs that realize the functions of these devices and systems.

### BRIEF DESCRIPTION OF DRAWINGS

Fig. 1 is an explanatory diagram illustrating a configuration of a medical apparatus according to a first embodiment.
Fig. 2 is an explanatory diagram of a magnetic sensor array and an ultrasonic probe.
Fig. 3 is an explanatory diagram illustrating a configuration of an ultrasonic probe.
Fig. 4 is a functional block diagram of a main control unit and a synthetic image generation unit.
Fig. 5 is a diagram that illustrates the processing of a device position information detection unit.
Figs. 6A and 6B are diagrams that illustrate a first example of a synthetic image.
Figs. 7A and 7B are diagrams that illustrate a second example of a synthetic image.
Figs. 8A and 8B are diagrams that illustrate a third example of a synthetic image.
Figs. 9A and 9B are diagrams that illustrate a modification of a synthetic image.
Fig. 10 is an explanatory diagram illustrating a configuration of a medical apparatus according to a second embodiment.
Fig. 11 is an explanatory diagram illustrating a configuration of an ultrasonic probe according to the second embodiment.
Fig. 12 is a functional block diagram of a main control unit and a synthetic image generation unit according to the second embodiment.
Fig. 13 is a diagram that illustrates the processing of a device position information detection unit according to the second embodiment.
Fig. 14 is a functional block diagram of a medical apparatus according to a third embodiment.
Figs. 15A and 15B are diagrams that illustrate a device position image according to the third embodiment.

### EMBODIMENTS OF THE INVENTION

### <First Embodiment>

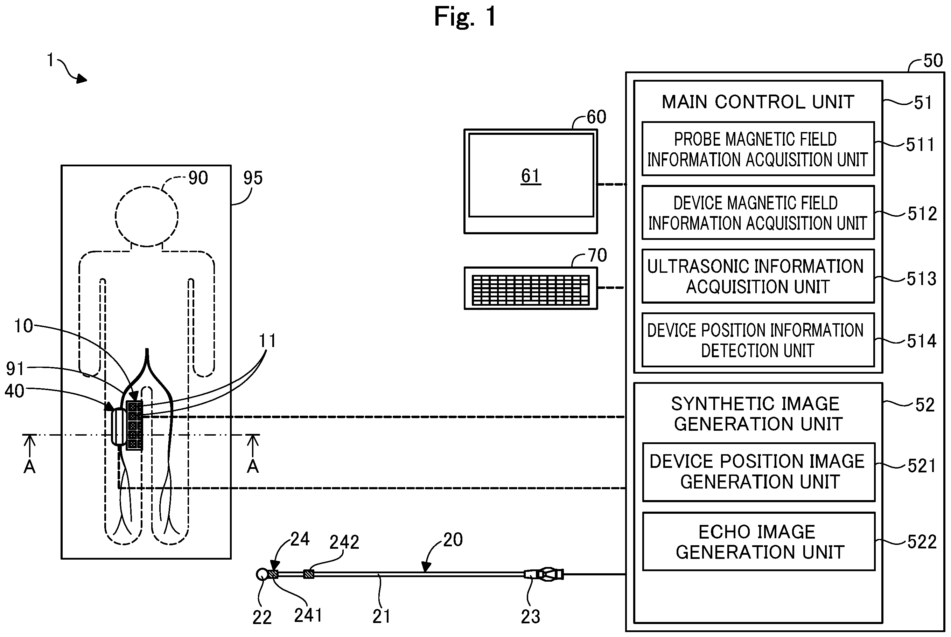

Fig. 1 is an explanatory diagram illustrating a configuration of a medical apparatus 1 according to a first embodiment. The medical apparatus 1 is a device used to treat or examine a living body (in this case, a human body) 90 that represents a treatment target, and includes a magnetic sensor array 10, a catheter 20, an ultrasonic probe 40, a computer 50, a display unit 60, and an operation unit 70. The medical apparatus 1 of the present embodiment generates a device position image that indicates the position of the catheter 20 inside a blood vessel from device magnetic field information and probe magnetic field information obtained from the magnetic sensor array 10, and an echo image of the blood vessel obtained from the ultrasonic probe 40. In the present embodiment, although a blood vessel is illustrated as an example of a biological lumen, in addition to the blood vascular system, the present device can also be applied to the lymphatic system, biliary system, urinary system, respiratory system, digestive system, secretory glands, reproductive organs, and the like.

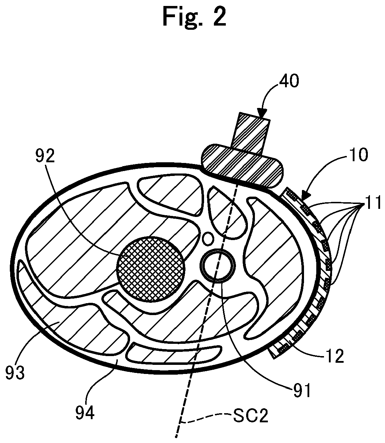

Fig. 2 is an explanatory diagram of the magnetic sensor array 10 and the ultrasonic probe 40. Fig. 2 represents a transverse cross-section taken along line A-A in Fig. 1. Fig. 2 illustrates, in addition to the magnetic sensor array 10 wrapped around the femoral region of the human body 90 and the ultrasonic probe 40 pressed against the femoral region, a bone 92, muscles 93, and fat 94 which surround the blood vessel 91.

The magnetic sensor array 10 is a device that detects the strength and orientation and the like of a magnetic field, namely the magnetic field generated by the catheter 20, and the magnetic field generated by the ultrasonic probe 40. The magnetic sensor array 10 includes a plurality of magnetic sensors 11 and a main body portion 12. The magnetic sensors 11 are devices that detect the strength and orientation of a magnetic field, and can be configured by a device such as a GSR (GHz-Spin-Rotation Sensor) sensor, a magnetoresistive effect device (MR), a magnetic impedance device (MI), or a superconducting quantum interference device (SUQUID). The magnetic sensors 11 are vertically and horizontally arranged side-by-side in a matrix form with respect to the main body portion 12. The main body portion 12 is a plate-shaped member having flexibility and elasticity, and is formed of, for example, rubber, a synthetic resin, a fabric, or the like.

As shown in Fig. 1 and Fig. 2, the magnetic sensor array 10 is wrapped around the femoral region of the human body 90. The magnetic sensor array 10 may be directly wrapped around the body surface (skin) of the human body 90, wrapped over clothing, wrapped over a lubricant, or wrapped over a space. Furthermore, the arrangement of the magnetic sensor array 10 can be arbitrarily changed, for example, and may be arranged on a table (bed) 95 on which the human body 90 lies, or wrapped around or attached to a part of the human body 90 other than the femoral region (such as the chest, waist, arms, neck, or head). In this case, the main body portion 12 of the magnetic sensor array 10 may be in the form of a garment, a hat, or a band. Moreover, the magnetic sensor array 10 may be three-dimensionally arranged with respect to the human body 90. In this case, for example, a plate-shaped first magnetic sensor array 10 may be arranged on the front of the human body 90, and a plate-shaped second magnetic sensor array 10 may be arranged on the back of the human body 90. In addition, the first and second magnetic sensor arrays 10 may be arranged in a state where the distance to the human body 90 is different on one side, on the front or the back of the human body 90.

The catheter 20 is inserted into the blood vessel 91 of the human body 90, and is a medical device used for treatment or examination. The catheter 20 includes a main body portion 21, a distal tip 22, a connector 23, and a device magnetic field generation unit 24. The main body portion 21 is a hollow member with an elongated outer shape. The distal tip 22 is a flexible member attached to the distal end of the main body portion 21. The connector 23 is a member provided on the proximal end of the main body portion 21, and is a member used when the operator grips the catheter 20.

The device magnetic field generation unit 24 functions as a "device magnetic field generation unit" for generating a magnetic field from the catheter 20. The device magnetic field generation unit 24 includes a first device magnetic field generation unit 241 and a second device magnetic field generation unit 242. The first device magnetic field generation unit 241 is a permanent magnet provided adjacent to the distal tip 22 at the distal end portion of the catheter 20. The second device magnetic field generation unit 242 is a permanent magnet provided at a predetermined distance from the first device magnetic field generation unit 241 on the proximal end side of the first device magnetic field generation unit 241. As a result of configuring the device magnetic field generation unit 24 with permanent magnets, the magnetic field strength generated by the device magnetic field generation unit 24 can be kept constant. Note that the device magnetic field generation unit 24 may be configured by electromagnets. In this case, the first device magnetic field generation unit 241 and the second device magnetic field generation unit 242 may be configured by conductive magnetic members (such as coils) connected to a current supply source. Also, the distal tip 22 and the first device magnetic field generation unit 241 may be integrally formed.

The configuration of the catheter 20 (medical device) shown in Fig. 1 is merely an example, and a medical device having an arbitrary configuration can be used as long as it includes the device magnetic field generation unit 24. For example, the distal tip 22 may be omitted, and the device may be configured as a so-called ablation catheter that generates plasma from the distal end. For example, instead of the catheter 20, a guide wire or the like provided with the device magnetic field generation unit 24 may be used.

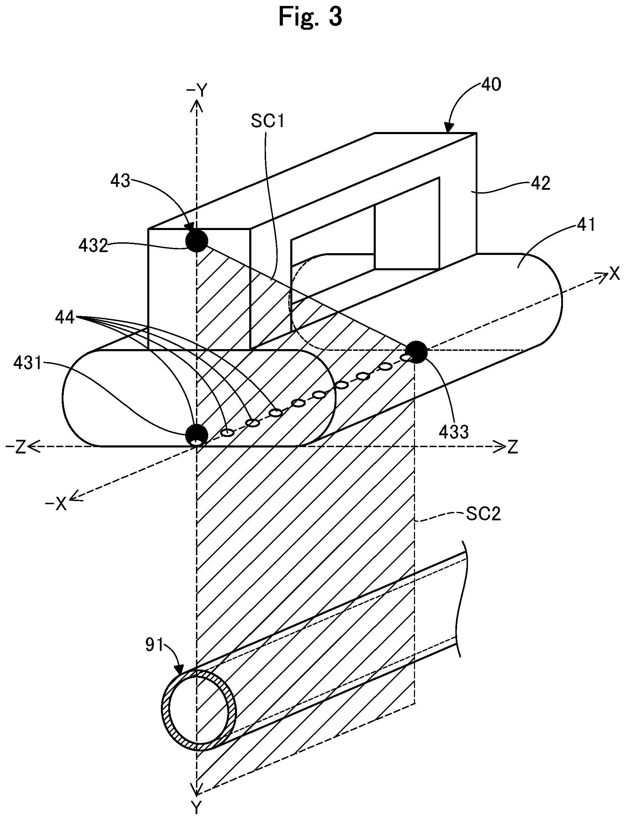

Fig. 3 is an explanatory diagram illustrating a configuration of the ultrasonic probe 40. Fig. 3 depicts X-, Y-, and Z-axes that are perpendicular to each another. The X-axis corresponds to the lengthwise direction of the ultrasonic probe 40 and the extending direction of the blood vessel 91. The Y-axis corresponds to the height direction of the ultrasonic probe 40 and the height direction of the blood vessel 91. The Z-axis corresponds to the width direction (shorter direction) of the ultrasonic probe 40 and the width direction of the blood vessel 91. The ultrasonic probe 40 is a device that intermittently irradiates the inside of the human body 90 including the blood vessel 91 with ultra-short ultrasonic pulses, and detects the strength of the reflected waves (echo). The ultrasonic probe 40 includes a main body portion 41, a handle portion 42, a probe magnetic field generation unit 43, and a plurality of ultrasonic sensors 44.

As shown in Fig. 2, the main body portion 41 is a housing that makes contact with the body surface (skin) of the human body 90. The handle portion 42 is a grip portion provided on the surface of the main body portion 41 opposite to the surface on which the ultrasonic sensors 44 are arranged, and is used by the operator to grip the ultrasonic probe 40. The handle portion 42 may be gripped by a robot arm or the like. Furthermore, the handle portion 42 may be omitted, and a robot arm may directly grip the main body portion 41. The main body portion 41 and the handle portion 42 can be formed of a resin material such as polyamide, polypropylene, polycarbonate, polyacetal, polyethersulfone, or the like.

The probe magnetic field generation unit 43 functions as a "magnetic field generation unit" for generating a magnetic field from the ultrasonic probe 40. The probe magnetic field generation unit 43 includes a first probe magnetic field generation unit 431, a second probe magnetic field generation unit 432, and a third probe magnetic field generation unit 433. The first probe magnetic field generation unit 431 is a permanent magnet embedded at one end in the lengthwise direction of the main body portion 41. The second probe magnetic field generation unit 432 is a permanent magnet embedded at one end in the lengthwise direction of the handle portion 42. The third probe magnetic field generation unit 433 is a permanent magnet embedded at another end in the lengthwise direction of the main body portion 41. Here, as shown in Fig. 3, the first probe magnetic field generation unit 431 and the second probe magnetic field generation unit 432 differ only in the positions in the Y-axis direction (that is, the height direction of the main body portion 41), and the positions in the X- and Z-axis directions are the same. On the other hand, the first probe magnetic field generation unit 431 and the third probe magnetic field generation unit 433 differ only in the position in the X-axis direction (that is, the lengthwise direction of the main body portion 41), and the position in the Y- and Z-axis directions is the same. Note that the phrase "same position" allows for manufacturing error, and the positions do not have to be exactly the same. Like the device magnetic field generation unit 24, the probe magnetic field generation unit 43 may also be configured by electromagnets.

The ultrasonic sensors 44 are ultrasonic probes (also referred to as ultrasonic transducers, piezoelectric bodies, ultrasonic transmitting/receiving elements, or ultrasonic elements) that transmit ultrasonic waves toward biological tissue inside the human body 90, such as the blood vessel 91, and receive reflected ultrasonic waves that are propagated through the biological tissue. In the example of Fig. 3, the ultrasonic sensors 44 are arranged side-by-side on a straight line connecting the first probe magnetic field generation unit 431 and the third probe magnetic field generation unit 433, on the surface of the main body portion 41 on the opposite side to the side on which the handle portion 42 is provided. This enables a plane SC 1 defined by the first to third probe magnetic field generation units 431 to 433 of the probe magnetic field generation unit 43 and a scanning plane (that is, the cross-section of the obtained ultrasonic echo image) SC2 of the ultrasonic sensors 44 to be on the same plane. The outside of each ultrasonic sensor 44 (that is, the surface on the positive Y-axis direction of the main body portion 41) may be covered by an acoustic matching layer, or an acoustic lens. The acoustic matching layer is a layer that adjusts the difference in acoustic impedance between the ultrasonic sensors 44 and the human body 90. The acoustic lens is a layer that focuses ultrasonic waves in the slice direction to improve resolution.

As shown in Fig. 2 and Fig. 3, the ultrasonic probe 40 is pressed against the femoral region of the human body 90 in a state where the extending direction of the blood vessel 91 is aligned with the direction in which the ultrasonic sensors 44 are arranged. At this time, the ultrasonic probe 40 is preferably pressed so that the scanning plane SC2 of the ultrasonic sensors 44 (that is, the cross-section of the obtained ultrasonic echo image) is positioned at the position where the diameter of the blood vessel 91 is the thickest (Fig. 3). The ultrasonic probe 40 may be directly pressed against the body surface (skin) of the human body 90, pressed over clothing, or pressed over a lubricant.

The description will be continued returning to Fig. 1. The display unit 60 is a liquid crystal display provided with a display screen 61. The display unit 60 functions as a "display unit" that displays an image generated by a synthetic image generation unit 52 described below. The display unit 60 may be configured by a display device other than a liquid crystal display (such as smart glasses or a projector). The operation unit 70 is a keyboard and a mouse for inputting information to the computer 50. The operation unit 70 may be configured by an input device other than a keyboard and a mouse (such as a microphone for acquiring audio input, a touch panel, or a foot switch).

The computer 50 is a device that controls the medical apparatus 1 as a whole, and is electrically connected to each of the magnetic sensor array 10, the ultrasonic probe 40, the display unit 60, and the operation unit 70. The computer 50 includes a CPU, a ROM, and a RAM (not illustrated), and realizes the functions of a main control unit 51 and a synthetic image generation unit 52 as a result of the CPU executing a computer program stored in the ROM.

The main control unit 51 exchanges information with the magnetic sensor array 10, the ultrasonic probe 40, the display unit 60, and the operation unit 70, and controls the medical apparatus 1 as a whole. The main control unit 51 includes a probe magnetic field information acquisition unit 511, a device magnetic field information acquisition unit 512, an ultrasonic information acquisition unit 513, and a device position information detection unit 514.

The probe magnetic field information acquisition unit 511 acquires probe magnetic field information from the magnetic sensor array 10. Here, the "probe magnetic field information" is information (an electrical signal) relating to the magnetic field generated from the ultrasonic probe 40, and is information that indicates the strength and orientation of the magnetic field associated with the probe magnetic field generation unit 43 of the ultrasonic probe 40. The probe magnetic field information is used to identify, as illustrated in Fig. 3, the plane SC1 defined by the probe magnetic field generation unit 43 and the scanning plane (that is, the cross-section of the obtained ultrasonic echo image) SC2 of the ultrasonic sensors 44 on the same plane as the plane SC 1.

The device magnetic field information acquisition unit 512 acquires device magnetic field information from the magnetic sensor array 10. Here, the "device magnetic field information" is information (an electrical signal) relating to the magnetic field generated from the catheter 20 inserted in the blood vessel 91 inside the human body 90, and is information that indicates the strength and orientation of the magnetic field associated with the device magnetic field generation unit 24 of the catheter 20. The device magnetic field information is used to identify the position of the catheter 20 inserted in the blood vessel 91.

The ultrasonic information acquisition unit 513 acquires ultrasonic information from the ultrasonic probe 40. Here, the "ultrasonic information" is information (an electrical signal) obtained by irradiating ultrasonic waves inside the human body 90, and is information that indicates the strength and orientation of the reflected waves (echo) detected by the ultrasonic probe 40. The ultrasonic information is used to generate the ultrasonic echo image.

The device position information detection unit 514 uses the device magnetic field information and the probe magnetic field information to detect the position of the catheter 20 inside the blood vessel 91. The details will be described later. Note that when the device magnetic field generation unit 24 and the probe magnetic field generation unit 43 are configured by electromagnets, the main control unit 51 may control the electric current supplied to the device magnetic field generation unit 24 and the probe magnetic field generation unit 43.

The synthetic image generation unit 52 generates an image and displays it on the display screen 61 of the display unit 60. The synthetic image generation unit 52 includes a device position image generation unit 521 and an echo image generation unit 522. The echo image generation unit 522 uses the ultrasonic information obtained by the ultrasonic information acquisition unit 513 to generate the ultrasonic echo image by a known method. The device position image generation unit 521 uses the position of the catheter 20 obtained by the device position information detection unit 514 and the ultrasonic echo image generated by the echo image generation unit 522 to generate the device position image. The details will be described later. The synthetic image generation unit 52 generates a synthetic image CI that combines the ultrasonic echo image and the device position image, and displays it on the display unit 60.

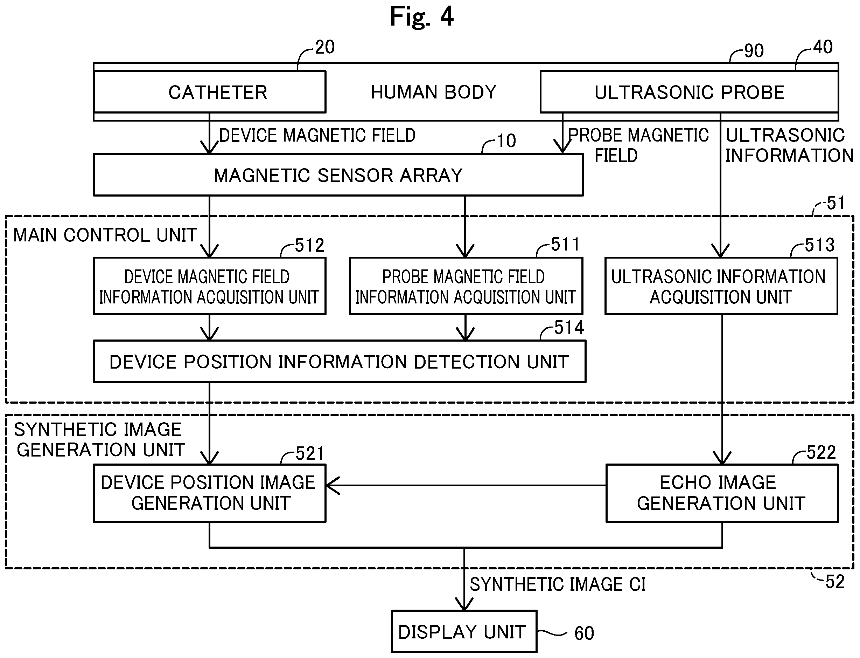

Fig. 4 is a functional block diagram of the main control unit 51 and the synthetic image generation unit 52. The device magnetic field information acquisition unit 512 of the main control unit 51 obtains device magnetic field information indicating the position of the device magnetic field generation unit 24 of the catheter 20 from the magnetic sensor array 10, and stores it in a storage unit (not illustrated) of the computer 50. Similarly, the probe magnetic field information acquisition unit 511 of the main control unit 51 acquires probe magnetic field information indicating the position of the probe magnetic field generation unit 43 of the ultrasonic probe 40 from the magnetic sensor array 10, and stores it in the storage unit of the computer 50.

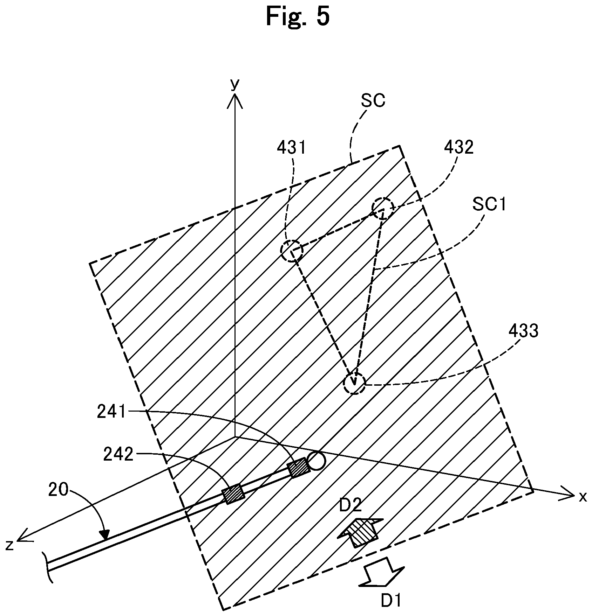

Fig. 5 is a diagram that illustrates the processing of the device position information detection unit 514. Fig. 5 depicts x-, y-, and z-axes that are perpendicular to each another. The x-, y-, and z-axes in Fig. 5 do not correspond to the X-, Y-, and Z-axes in Fig. 3. The device position information detection unit 514 of the main control unit 51 uses the probe magnetic field information acquired from the magnetic sensor array 10 and the device magnetic field information to detect the position of the catheter 20 inside the blood vessel 91. First, the device position information detection unit 514 uses the probe magnetic field information to define a reference plane SC on the same plane as a plane SC1 on which the probe magnetic field generation unit 43 (first to third probe magnetic field generation units 431 to 43) is arranged. As illustrated in Fig. 3, the reference plane SC is also on the same plane as the scanning plane (the cross-section of the obtained ultrasonic echo image) SC2 of the ultrasonic sensors 44. Then, the device position information detection unit 514 uses the device magnetic field information to identify the positional relationship between the reference plane SC and the catheter 20. Specifically, the device position information detection unit 514 uses the device magnetic field information to identify whether the first device magnetic field generation unit 241 of the catheter 20 is positioned on the reference plane SC, positioned on one side D 1 of the reference plane SC, or positioned on another side D2 of the reference plane SC. Similarly, the device position information detection unit 514 uses the device magnetic field information to identify whether the second device magnetic field generation unit 242 of the catheter 20 is positioned on the reference plane SC, positioned on the one side D1 of the reference plane SC, or positioned on the another side D2 of the reference plane SC.

The ultrasonic information acquisition unit 513 of the main control unit 51 acquires ultrasonic information for generating the ultrasonic echo image from the ultrasonic probe 40, and stores it in a storage unit of the computer 50.

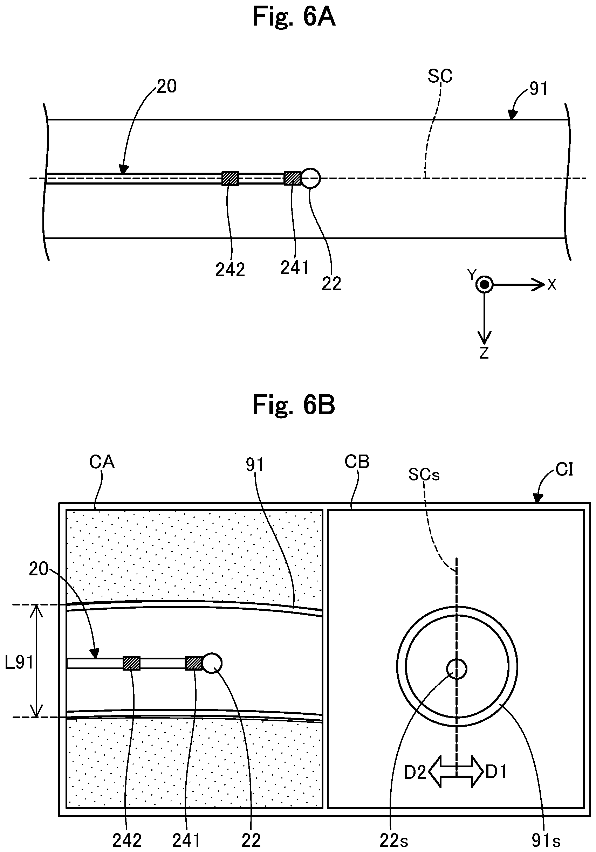

Figs. 6A and 6B are respectively a diagram that illustrates a first example of a synthetic image CI. Fig. 6A illustrates the positional relationship between the blood vessel 91, the catheter 20, and the reference plane SC of the ultrasonic probe 40. The X-, Y-, and Z-axes in Fig. 6A each correspond to the X-, Y-, and Z-axes in Fig. 3, and do not correspond to the x-, y-, and z-axes in Fig. 5. In the example of Fig. 6A, the reference plane SC of the ultrasonic probe 40 is provided at the position at which height of the blood vessel 91 (in other words, the diameter in the Y-axis direction of the blood vessel 91) is the maximum, and the catheter 20 is advancing inside the blood vessel 91 on the reference plane SC.

Fig. 6B illustrates the synthetic image CI displayed on the display unit 60. The echo image generation unit 522 uses the ultrasonic information in the storage unit to generate an ultrasonic echo image CA, which is a two-dimensional image with a shading gradation that corresponds to the intensity of the reflected waves at the ultrasonic sensors 44. As shown in Fig. 6B, the ultrasonic echo image CA includes the blood vessel 91 and the catheter 20 in the scanning plane SC2 of the ultrasonic sensors 44, which is the same plane as the reference plane SC. The ultrasonic echo image CA also includes other body tissues surrounding the blood vessel 91, such as muscle and fat.

The device position image generation unit 521 uses the ultrasonic echo image CA and the positional relationship identified by the device position information detection unit 514 (the positional relationship between the reference plane SC and the catheter 20) to generate a device position image CB. The device position image CB of the present embodiment is an image in which an image SCs indicating the position of the reference plane SC that traverses the blood vessel 91 and an image 22s indicating the position of the distal end portion of the catheter 20 are superimposed on an image 91s representing the transverse cross-section of the blood vessel 91.

Specifically, the device position image generation unit 521 firstly performs image analysis of the ultrasonic echo image CA to determine the size of the blood vessel 91 occupying the imaging range of the ultrasonic echo image CA, and the size of the blood vessel 91 relative to the catheter 20, and then calculates the diameter L91 of the blood vessel 91. The diameter L91 can be any of the outer diameter, the inner diameter, or a combination of the outer diameter and the inner diameter of the portion of the blood vessel 91 that intersects the reference plane SC. Then, the device position image generation unit 521 generates a blood vessel model 91s that represents the transverse cross-section of a blood vessel 91 having the calculated diameter L91, and arranges it on the device position image CB. A line segment SCs that represents the reference plane SC is drawn on the blood vessel model 91s at the position where the height (in other words, the diameter in the Y-axis direction) is the maximum. Then, the device position image generation unit 521 draws a distal end model 22s that represents the distal tip 22 at the position corresponding to the positional relationship identified by the device position information detection unit 514. Specifically, when the first device magnetic field generation unit 241 is positioned on the reference plane SC, the device position image generation unit 521 draws the distal end model 22s overlaid on the line segment SCs. Furthermore, the device position image generation unit 521 draws the distal end model 22s on the one side D1 of the line segment SCs when the first device magnetic field generation unit 241 is positioned on the one side D1 of the reference plane SC, and draws the distal end model 22s on the another side D2 of the line segment SCs when the first device magnetic field generation unit 241 is positioned on the another side D2 of the reference plane SC.

The synthetic image generation unit 52 generates the synthetic image CI, in which the ultrasonic echo image CA generated by the echo image generation unit 522 and the device position image CB generated by the device position image generation unit 521 are laterally arranged, and displays it on the display unit 60. When the synthetic image CI shown in Fig. 6B is being displayed, the operator refers to the synthetic image CI while advancing the catheter 20 as is. Note that the ultrasonic echo image CA and the device position image CB may be vertically arranged in the synthetic image CI, and may be arranged with different sizes in the synthetic image CI. For example, in the synthetic image CI, one of the images (such as the device position image CB) may be displayed relatively large as a primary image, and the other image (such as the ultrasonic echo image CA) may be displayed relatively small as a secondary image. In this case, the primary image and the secondary image may be switchable by the operator.

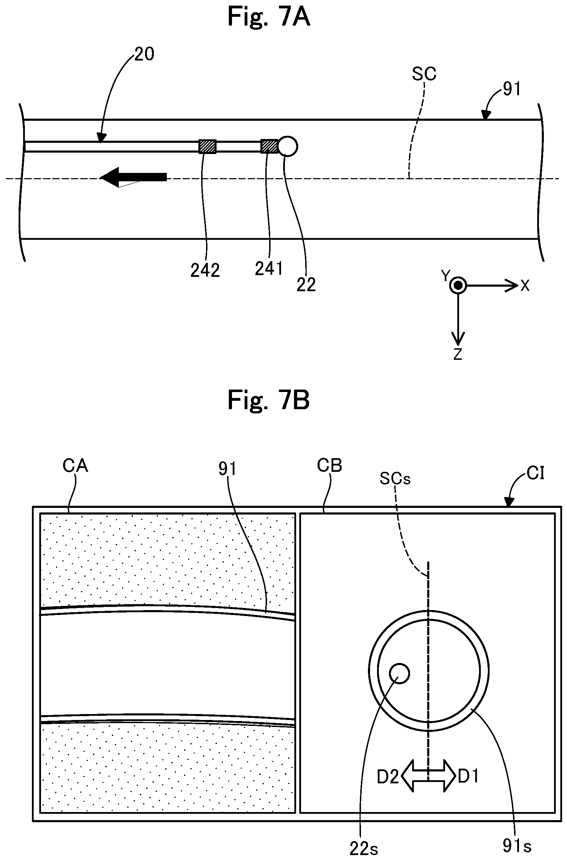

Figs. 7A and 7B are respectively a diagram that illustrates a second example of a synthetic image CI. The configuration in Figs. 7A and 7B is the same as in Figs. 6A and 6B. In the example of Fig. 7A, the catheter 20 inside the blood vessel 91 is advancing on the another side of the reference plane SC, and is not on the reference plane SC. In this case, as shown in Fig. 7B, the ultrasonic echo image CA in the synthetic image CI contains only the blood vessel 91, and does not contain the catheter 20. This is because the catheter 20 is not on the reference plane SC, that is, the scanning plane SC2 of the ultrasonic sensors 44. Furthermore, in the device position image CB of the synthetic image CI, the distal end model 22s is drawn on the another side D2 of the line segment SCs representing the reference plane SC of the blood vessel model 91s. When the synthetic image CI shown in Fig. 7B is being displayed, the operator pulls the catheter 20 back in the direction of the black arrow shown in Fig. 7A, and refers to the synthetic image CI while operating the catheter 20 such that it becomes positioned on the reference plane SC.

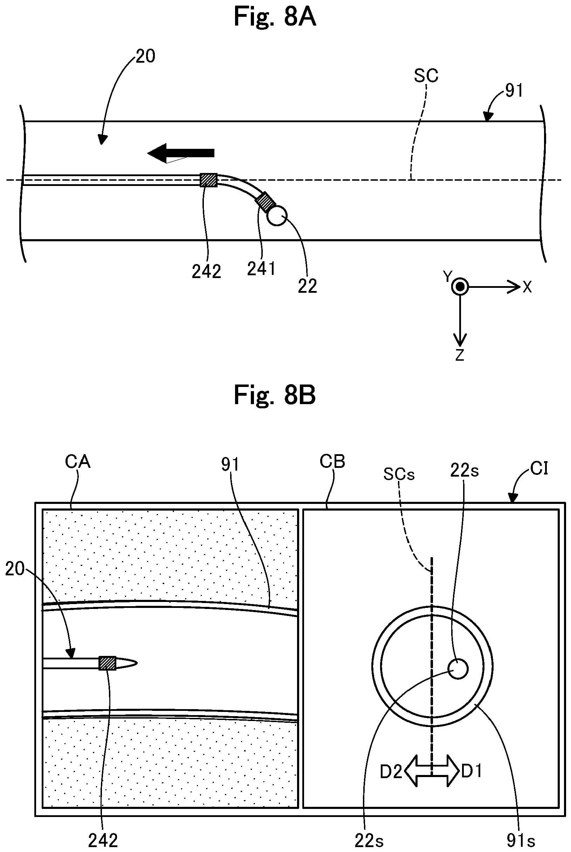

Figs. 8A and 8B are respectively a diagram that illustrates a third example of a synthetic image CI. The configuration in Figs. 8A and 8B is the same as in Figs. 6A and 6B. In the example of Fig. 8A, a portion of the distal end side of the catheter 20 (in the illustrated example, the distal tip 22 and a portion near the first device magnetic field generation unit 241) has advanced inside the blood vessel 91 on the one side of the reference plane SC, and is not on the reference plane SC. On the other hand, a portion on the proximal end side of the catheter 20 (in the illustrated example, the portion on the proximal end side of the second device magnetic field generation unit 242) is advancing on the reference plane SC. In this case, as shown in Fig. 8B, the ultrasonic echo image CA in the synthetic image CI contains the blood vessel 91, and the portion on the proximal end side of the catheter 20. Furthermore, in the device position image CB of the synthetic image CI, the distal end model 22s is drawn on the one side D 1 of the line segment SCs representing the reference plane SC of the blood vessel model 91s. This is because the first device magnetic field generation unit 241 is positioned on the one side of the reference plane SC. When the synthetic image CI shown in Fig. 8B is being displayed, the operator pulls the catheter 20 back in the direction of the black arrow shown in Fig. 8A, and refers to the synthetic image CI while correcting the curvature of the distal end portion of the catheter 20.

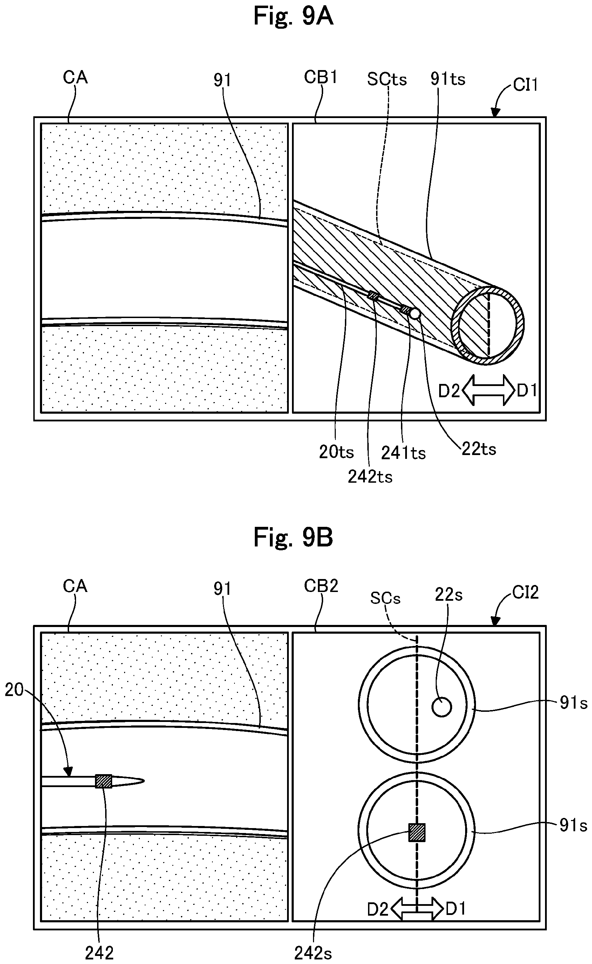

Figs. 9A and 9B are respectively a diagram that illustrates a modification of a synthetic image CI. Fig. 9A shows the synthetic image CI1, which is a first modification of the synthetic image CI. The synthetic image CI1 is an image in which the ultrasonic echo image CA illustrated in Fig. 6B and a device position image CB 1 described next have been combined. The device position image CB1 is an image in which a plane SCts representing the reference plane SC that traverses the blood vessel 91 and a three-dimensional image 20ts of the distal end portion of the catheter 20 are superimposed on a three-dimensional image 91ts of the blood vessel 91. The device position image generation unit 521 generates, from the diameter L91 of the blood vessel 91 obtained from the ultrasonic echo image CA, a three-dimensional image 91ts of a blood vessel 91 having the diameter L91, and arranges it on the device position image CB1. The three-dimensional image 91ts of the blood vessel 91 has the plane SCts representing the reference plane SC drawn at the position where the height is the maximum. Then, the device position image generation unit 521 draws the catheter 20 at the position corresponding to the positional relationship identified by the device position information detection unit 514. Specifically, the device position image generation unit 521 draws a distal end model 22ts and a model 241ts of the first device magnetic field generation unit 241 at the positions corresponding to the positional relationship between the first device magnetic field generation unit 241 and the reference plane SC. Furthermore, the device position image generation unit 521 draws a model 242ts of the second device magnetic field generation unit 242 at the position corresponding to the positional relationship between the second device magnetic field generation unit 242 and the reference plane SC.

Fig. 9B shows the synthetic image CI2, which is a second modification of the synthetic image CI. The synthetic image CI2 is an image in which the ultrasonic echo image CA illustrated in Fig. 6B and a device position image CB2 described next have been combined. The device position image CB2 is configured by two vertical panels, and the upper panel is the same as Fig. 6B. The lower panel of the device position image CB2 is an image in which an image SCs indicating the position of the reference plane SC that traverses the blood vessel 91 and an image 242s indicating the position of the second device magnetic field generation unit 242 of the catheter 20 are superimposed on an image 91s representing the transverse cross-section of the blood vessel 91. The synthetic image generation unit 52 and the device position image generation unit 521 may generate the synthetic image CI1 illustrated in Fig. 9A or the synthetic image CI2 illustrated in Fig. 9B, and display it on the display unit 60.

As described above, the medical apparatus 1 according to the first embodiment includes the device position image generation unit 521 that generates the device position image CB that indicates the position of the catheter 20 (medical device) inside the blood vessel 91 from the ultrasonic echo image CA and device magnetic field information. This allows the operator such as a physician to confirm the position of the catheter 20 inside the blood vessel 91 from the device position image CB, which enables the catheter 20 inside the blood vessel 91 to be easily moved to the intended position (that is, the position at which the height of the blood vessel 91 is the maximum). As a result, the effort and time required for the procedure can be reduced, and the accuracy of the procedure can be improved.

Furthermore, the medical apparatus 1 according to the first embodiment includes the ultrasonic probe 40 provided with the probe magnetic field generation unit 43 (magnetic field generation unit), and the probe magnetic field information acquisition unit 511 that acquires probe magnetic field information relating to the magnetic field generated from the ultrasonic probe 40. As a result, the medical apparatus 1 uses the probe magnetic field information to align the scanning plane SC2 of the ultrasonic sensors 44, that is, the longitudinal cross-section SC2 of the blood vessel 91 (in other words, the reference plane SC that traverses the blood vessel) appearing in the ultrasonic echo image CA and the device magnetic field information (Fig. 5), which then enables the position of the catheter 20 inside the blood vessel 91 to be identified from the device magnetic field information, and the device position image CB to be generated. Furthermore, the device position image CB is an image that indicates whether the catheter 20 is positioned, with respect to the longitudinal cross-section of the blood vessel 91 (the reference plane SC that traverses the blood vessel 91) appearing in the ultrasonic echo image CA, on the one side D1 or positioned on the another side D2 of the longitudinal cross-section. As a result, the operator can check the device position image CB to easily grasp the direction that the catheter 20 should be advanced.

Further, according to the examples of Figs. 6A and 6B to Figs. 8A and 8B, and Fig. 9B, the device position image generation unit 521 generates the device position images CB and CB2, in which the image SCs indicating the position of the longitudinal cross-section of the blood vessel 91, and the image 22s indicating the position of the catheter 20 (medical device) are superimposed on the image 91s representing the transverse cross-section of the blood vessel 91. As a result, the operator can check the device position image CB to intuitively grasp the position of the catheter 20 inside the blood vessel 91, and the direction that the catheter 20 should be advanced.

Further, according to the example in Fig. 9A, the device position image generation unit 521 generates the device position image CB1, in which the plane SCts representing the longitudinal cross-section of the blood vessel 91, and the three-dimensional images 22ts, 241ts and 242ts of the distal end portion of the catheter 20 (medical device) are superimposed on the three-dimensional image 91ts of the blood vessel 91. As a result, the operator can check the device position image CB1 to even more intuitively grasp the position of the catheter 20 inside the blood vessel 91, and the direction that the catheter 20 should be advanced.

In addition, the medical apparatus 1 according to the first embodiment includes the synthetic image generation unit 52 that generates the synthetic image CI by combining the ultrasonic echo image CA and the device position image CB. As a result, the operator is capable of checking the ultrasonic echo image CA and the device position image CB at the same time while performing the procedure, which enables the effort and time required for the procedure to be further reduced, while also further improving the accuracy of the procedure. Furthermore, as illustrated in Figs. 7A and 7B and Figs. 8A and 8B, even when a displacement in the position of the ultrasonic probe 40 and the catheter 20 (medical device) causes the catheter 20 to no longer be confirmed in the ultrasonic echo image CA, the operator is able to easily grasp the direction that the catheter 20 should be advanced by checking the device position image CB, such that the catheter 20 can easily be returned to the intended position (such as on the longitudinal cross-section of the blood vessel 91 that appears in the ultrasonic echo image CA).

### <Second Embodiment>

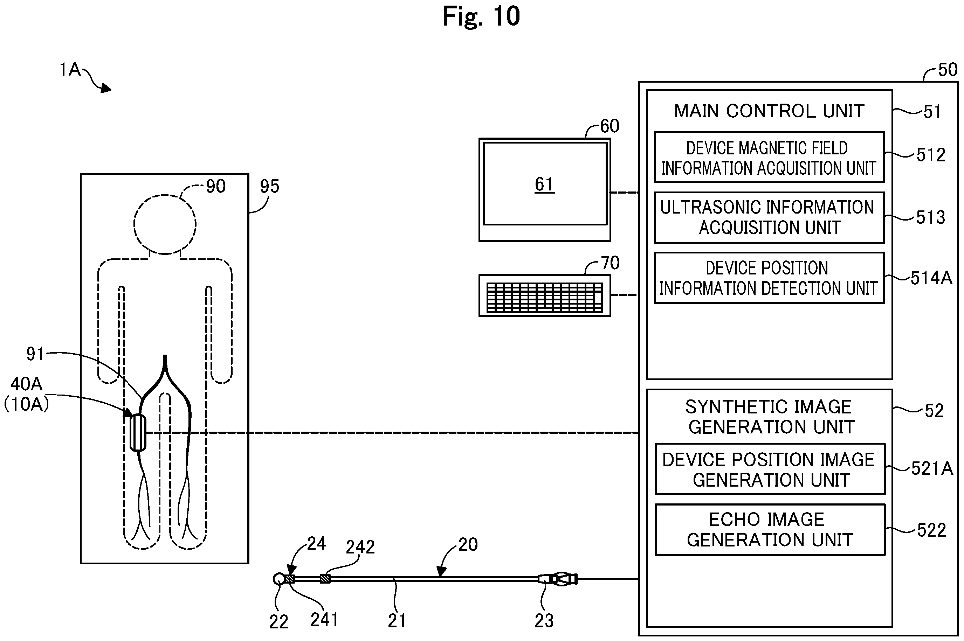

Fig. 10 is an explanatory diagram illustrating a configuration of a medical apparatus 1A according to a second embodiment. The medical apparatus 1A according to the second embodiment generates the same synthetic image CI as the first embodiment by using an ultrasonic probe 40A in which the magnetic sensor array 10 and the ultrasonic probe 40 described in the first embodiment are integrated. Of the configuration of the first embodiment, the medical apparatus 1A includes the ultrasonic probe 40A in place of the magnetic sensor array 10 and the ultrasonic probe 40, does not include the probe magnetic field information acquisition unit 511, includes a device position information detection unit 514A in place of the device position information detection unit 514, and includes a device position image generation unit 521A in place of the device position image generation unit 521.

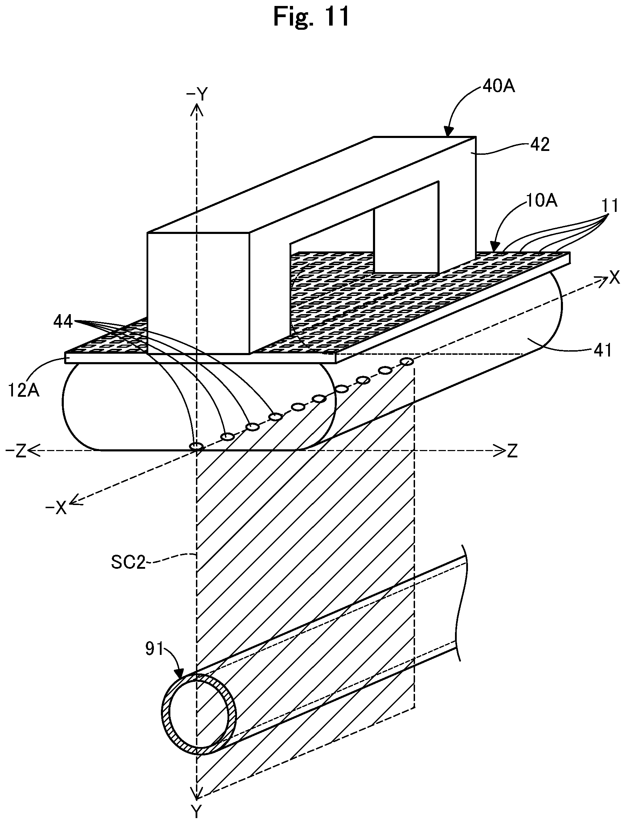

Fig. 11 is an explanatory diagram illustrating a configuration of the ultrasonic probe 40A according to the second embodiment. Fig. 11 illustrates the same X-, Y-, and Z-axes as Fig. 3. The ultrasonic probe 40A has the magnetic sensor array 10A fixed to a surface of the main body portion 41 on the opposite side to the side having the surface on which the ultrasonic sensors 44 are arranged. The magnetic sensor array 10A includes a plurality of magnetic sensors 11 and a substantially rectangular main body portion 12A. The magnetic sensors 11 have the same configuration as in the first embodiment, and are vertically and horizontally arranged side-by-side in a matrix form with respect to the main body portion 12A. In this manner, in the ultrasonic probe 40A according to the second embodiment, the positional relationship between the ultrasonic sensors 44 and the magnetic sensors 11 is fixed, while the probe magnetic field generation unit 43 described in the first embodiment is not provided. Like the first embodiment, the ultrasonic probe 40A is pressed against the femoral region of the human body 90 in a state where the extending direction of the blood vessel 91 is aligned with the direction in which the ultrasonic sensors 44 are arranged. For example, the main body portion 12A can be formed of rubber or a synthetic resin or the like.

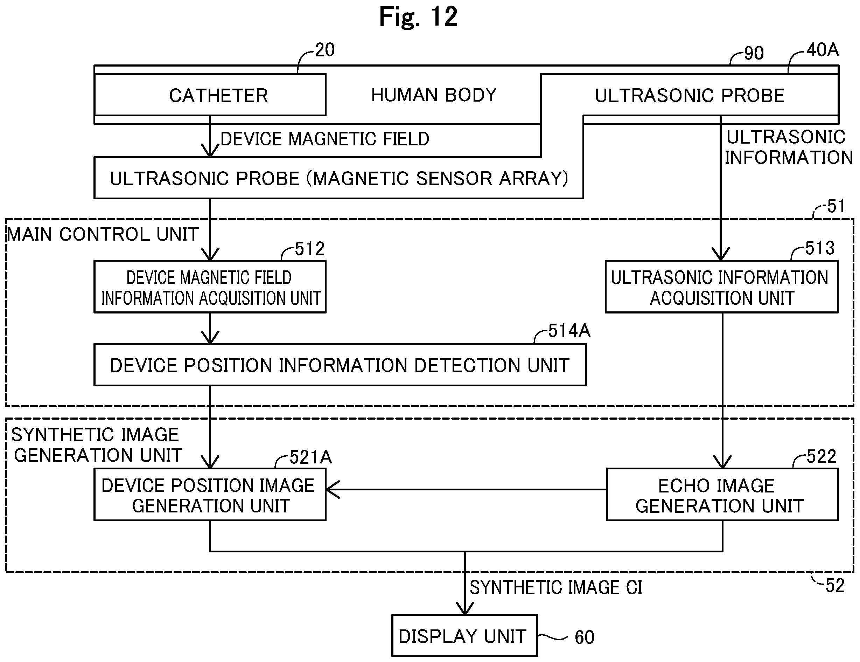

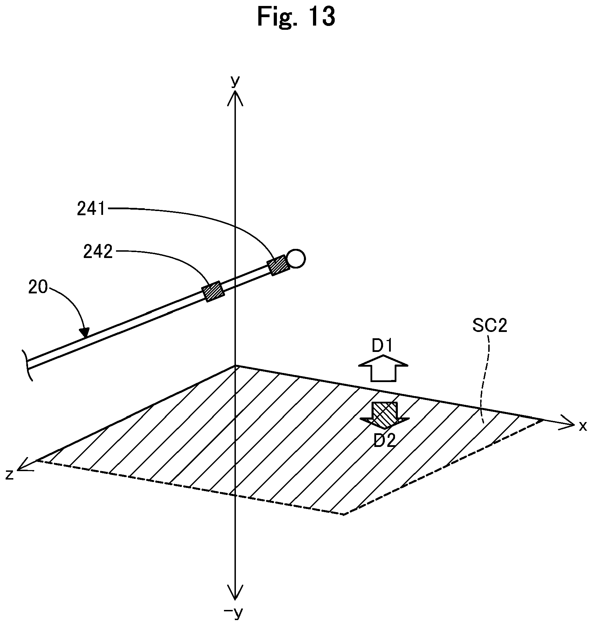

Fig. 12 is a functional block diagram of the main control unit 51 and the synthetic image generation unit 52 according to the second embodiment. Fig. 13 is a diagram that illustrates the processing of the device position information detection unit 514A according to the second embodiment. The x-, y-, and z-axes in Fig. 13 do not correspond to the X-, Y-, and Z-axes in Fig. 11. The device position information detection unit 514A according to the second embodiment detects the position of the catheter 20 inside the blood vessel 91 using only the device magnetic field information acquired from the magnetic sensor array 10A of the ultrasonic probe 40A (that is, it does not use the probe magnetic field information described in the first embodiment). As illustrated in Fig. 11, in the ultrasonic probe 40A of the present embodiment, the positional relationship between the ultrasonic sensors 44 and the magnetic sensors 11 is fixed. As a result, by storing the known positional relationship between the ultrasonic sensors 44 and the magnetic sensors 11 in advance, the device position information detection unit 514A is capable of identifying the positional relationship between the scanning plane (the cross-section of the obtained ultrasonic echo image) SC2 of the ultrasonic sensors 44 of the ultrasonic probe 40A and the catheter 20 by using only the device magnetic field information. Specifically, the device position information detection unit 514A can use the device magnetic field information to identify whether the first device magnetic field generation unit 241 of the catheter 20 is positioned on the reference plane SC2, positioned on the one side D1 of the reference plane SC2, or positioned on the another side D2 of the reference plane SC2. The same is true for the second device magnetic field generation unit 242 of the catheter 20.

The device position image generation unit 521A uses the ultrasonic echo image CA and the positional relationship identified by the device position information detection unit 514A (the positional relationship between the reference plane SC2 and the catheter 20) to generate the device position image CB. The device position image CB according to the second embodiment is the same as that of the first embodiment illustrated in Figs. 6A and 6B to Figs. 9A and 9B except in that it is generated using the known positional relationship between the ultrasonic sensors 44 and the magnetic sensor 11.

As described above, various modifications are possible to the configuration of the medical apparatus 1A, and the synthetic image CI may be generated by using the ultrasonic probe 40A in which the magnetic sensor array 10 and the ultrasonic probe 40 are integrated. Furthermore, the configuration of the ultrasonic probe 40A illustrated in Fig. 11 is merely an example, and the magnetic sensors 11 may be built into the ultrasonic probe 40A in an arbitrary form. The same effects as those of the first embodiment can still be obtained in this case.

Moreover, according to the medical apparatus 1A of the second embodiment, the medical apparatus 1A includes the ultrasonic probe 40A, which is provided with the magnetic sensors 11 having a fixed positional relationship with the ultrasonic sensors 44. That is, according to the configuration of the second embodiment, because the positional relationship between the ultrasonic sensors 44 and the magnetic sensors 11 of the ultrasonic probe 40A is already known, the medical apparatus 1A is capable of identifying the position of the catheter 20 (medical device) inside the blood vessel 91 from the device magnetic field information, and generating the device position image CB without aligning the longitudinal cross-section of the blood vessel 91 (the reference plane SC that traverses the blood vessel 91) appearing in the ultrasonic echo image CA and the device magnetic field information. Furthermore, compared to the first embodiment, because the distance from the magnetic sensors 11 and the ultrasonic sensors 44 to the blood vessel 91 into which the catheter 20 is inserted can be made shorter, the detection accuracy of the magnetic sensors 11 and the ultrasonic sensors 44 can be improved, and the reliability of the position information of the catheter 20 obtained by the magnetic sensor 11 can be improved. In addition, the device position image CB is an image that indicates whether the catheter 20 is positioned, with respect to the longitudinal cross-section of the blood vessel 91 appearing in the ultrasonic echo image CA, on the one side or positioned on the another side of the longitudinal cross-section. As a result, the operator can check the device position image CB to easily grasp the direction that the catheter 20 should be advanced.

### <Third Embodiment>

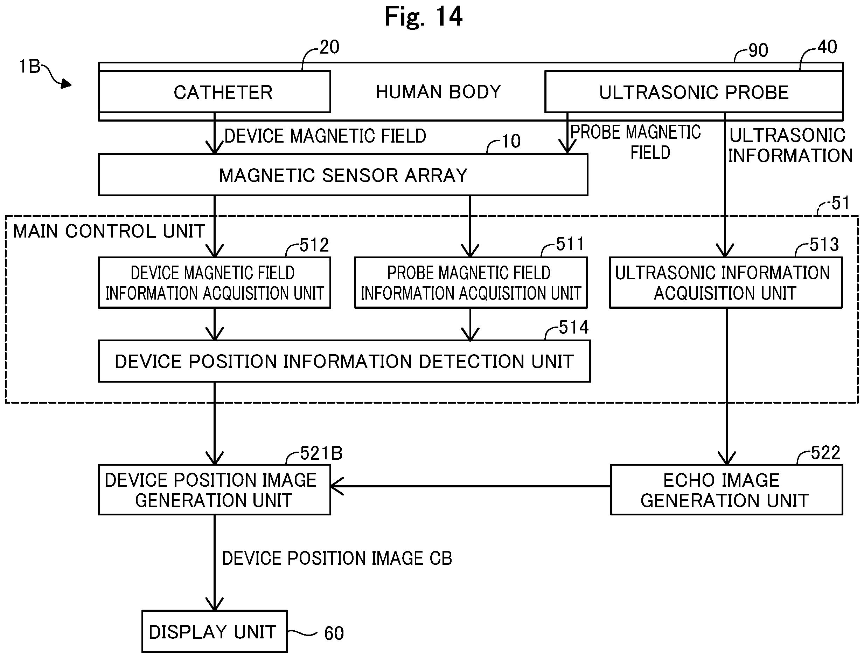

Fig. 14 is a functional block diagram of a medical apparatus 1B according to a third embodiment. The medical apparatus 1B according to the third embodiment does not generate or display the synthetic image CI described in the first embodiment. Of the configuration described in the first embodiment, the medical apparatus 1B does not include the synthetic image generation unit 52, and also includes a device position image generation unit 521B in place of the device position image generation unit 521.

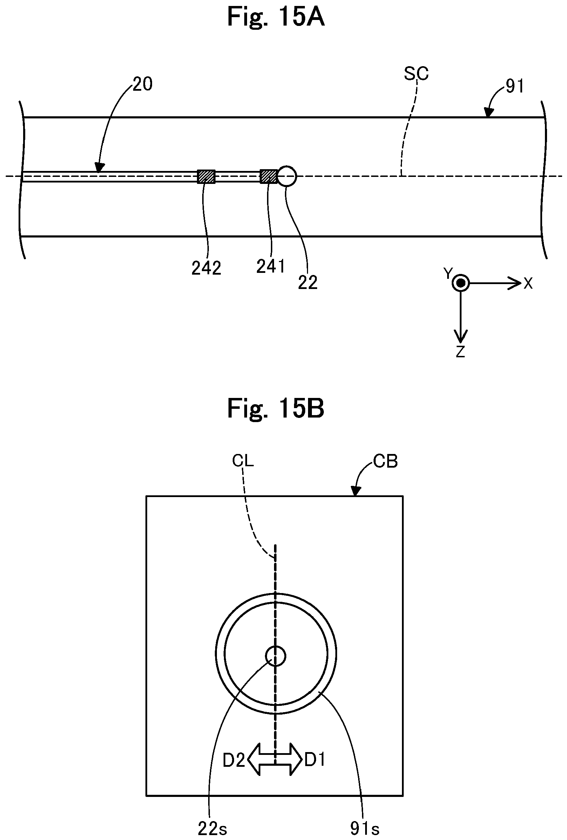

Figs. 15A and 15B are respectively a diagram that illustrates a device position image CB according to the third embodiment. The configuration in Figs. 15A and 15B is the same as in Fig. 6A and 6B. In the example of Fig. 15A, like in Fig. 6A, the reference plane SC of the ultrasonic probe 40 is provided at the position at which height of the blood vessel 91 is the maximum, and the catheter 20 is advancing inside the blood vessel 91 on the reference plane SC. In this case, as shown in Fig. 15B, the device position image generation unit 521B uses the ultrasonic echo image CA and the positional relationship identified by the device position information detection unit 514 (the positional relationship between the reference plane SC and the catheter 20) to generate the device position image CB. Then, the device position image generation unit 521B displays the generated device position image CB on the display unit 60.

As described above, various modifications are possible to the configuration of the medical apparatus 1B, and it is possible for the display unit 60 to display only the device position image CB rather than displaying the ultrasonic echo image CA or the synthetic image CI. The same effects as those of the first embodiment can still be obtained in this case.

### <Modifications of Present Embodiments>

In the embodiments described above, part of the configuration realized by hardware may be replaced with software, or conversely, part of the configuration realized by software may be replaced with hardware. Furthermore, the disclosed embodiments are not limited to the embodiments described above and may be carried out in various aspects without departing from the spirit thereof, and for example, the following modifications are possible.

### [First Modification]

In the first to third embodiments, the configurations of the medical apparatuses 1, 1A and 1B have been illustrated. However, various modifications are possible to the configuration of the medical apparatus 1. For example, in the medical apparatus 1, at least a portion of magnetic sensor array 10, the ultrasonic probe 40, the computer 50, the display unit 60, and the operation unit 70 may be configured as an integrated device. For example, the medical apparatus 1 may be provided with another device such as a CT device, an MRI device, an electrocardiograph, or an X-ray imaging device.

### [Second Modification]

In the first to third embodiments described above, an example of the device position image CB generated by the device position image generation units 521, 521A and 521B, and an example of the synthetic image CI generated by the synthetic image generation unit 52 have been illustrated. However, various modifications are possible to these images. For example, although the device position image CB was assumed to include the blood vessel model 91s, the line segment SCs indicating the position of the reference plane SC, and the distal end model 22s as a representation of the position of the catheter 20 (medical device) inside the blood vessel 91, at least some of these may be omitted or modified. For example, the device position image CB may be configured by the blood vessel model 91s and the distal end model 22s, and may not include the line segment SCs. For example, the device position image CB may be configured by the line segment SCs and the distal end model 22s, and may not include the blood vessel model 91s. For example, instead of the distal end model 22s, an image representing the first device magnetic field generation unit 241 may be used, or an image representing the main body portion 21 may be used. For example, instead of the distal end model 22s, an arbitrary character, symbol, graphic, or the like may be used.

For example, the synthetic image CI may include arbitrary images, such as images obtained by other devices provided in the medical apparatus 1 (such as a CT device, an MRI device, an electrocardiograph, or an X-ray imaging device), images generated based on information obtained by other devices (such as a three-dimensional model of an organ), images acquired from an external storage medium, and images obtained from a network. For example, the images included in the synthetic image CI (the ultrasonic echo image CA, the device position image CB, and the other images mentioned above) may be displayed/not displayed according to a selection made by the user. In this case, the synthetic image CI may include a window for selecting the images to be displayed.

### [Third Modification]

The configurations of the medical apparatuses 1, 1A and 1B according to the first to third embodiments described above, and the configurations of the first and second modifications described above may be appropriately combined. For example, in the configuration using the ultrasonic probe 40A described in the second embodiment, the device position image CB described in the third embodiment may be displayed.

The aspects have been described above based on the embodiments and the modifications, but the embodiments of the aspects described above are provided to facilitate understanding the aspects and not to limit the aspects. The aspects may be modified and improved without departing from the spirit of the aspects and the scope of the claims, and equivalents thereof are included in the aspects. Further, unless the technical features are described as essential in the present specification, they may be omitted as appropriate.

### DESCRIPTION OF REFERENCE NUMERALS

1, 1A, 1B Medical apparatus
10, 10A Magnetic sensor array
11 Magnetic sensor
12, 12A Main body portion
20 Catheter
21 Main body portion
22 Distal tip
23 Connector
24 Device magnetic field generation unit
40, 40A Ultrasonic probe
41 Main body portion
42 Handle portion
43 Probe magnetic field generation unit
44 Ultrasonic sensor
50 Computer
51 Main control unit
52 Synthetic image generation unit
60 Display unit
61 Display screen
70 Operation unit
90 Human body
91 Blood vessel
241 First device magnetic field generation unit
242 Second device magnetic field generation unit
431 First probe magnetic field generation unit
432 Second probe magnetic field generation unit
433 Third probe magnetic field generation unit
511 Probe magnetic field information acquisition unit
512 Device magnetic field information acquisition unit
513 Ultrasonic information acquisition unit
514, 514A Device position information detection unit
515 Operation information acquisition unit
516 Position estimation unit
521, 521A, 521B Device position image generation unit
522 Echo image generation unit

## Claims

1. A medical apparatus comprising:
an ultrasonic information acquisition unit that acquires in-vivo ultrasonic information of inside of a living body obtained from an ultrasonic probe that has an ultrasonic sensor and emits ultrasonic waves inside the living body;
a device magnetic field information acquisition unit that obtains device magnetic field information relating to a magnetic field generated from a medical device inserted in a blood vessel inside the living body;
an echo image generation unit that generates an ultrasonic echo image of the blood vessel from the ultrasonic information; and
a device position image generation unit that generates a device position image indicating a position of the medical device inside the blood vessel, based on the ultrasonic echo image and the device magnetic field information.

2. The medical apparatus according to claim 1, further comprising:
the ultrasonic probe provided with a magnetic field generation unit; and
a probe magnetic field information acquisition unit that acquires probe magnetic field information relating to a magnetic field generated from the ultrasonic probe, wherein
the device position image generation unit generates, with respect to a reference plane that traverses the blood vessel defined by the probe magnetic field information, the device position image that indicates whether the medical device is positioned on one side or positioned on another side of the reference plane.

3. The medical apparatus according to claim 1, further comprising
the ultrasonic probe provided with a magnetic sensor having a fixed positional relationship with the ultrasonic sensor, wherein
the device position image generation unit generates, with respect to a reference plane that traverses the blood vessel, the device position image that indicates whether the medical device is positioned on one side or positioned on another side of the reference plane using the positional relationship between the ultrasonic sensor and the magnetic sensor.

4. The medical apparatus according to claim 2 or 3, wherein
the device position image generation unit generates the device position image, in which an image indicating a position of the reference plane that traverses the blood vessel, and an image indicating a position of the medical device are superimposed on an image representing a transverse cross-section of the blood vessel.

5. The medical apparatus according to claim 2 or 3, wherein
the device position image generation unit generates the device position image, in which a plane representing the reference plane that traverses the blood vessel, and a three-dimensional image of a distal end portion of the medical device are superimposed on a three-dimensional image of the blood vessel.

6. The medical apparatus according to any one of claims 1 to 5, further comprising
a synthetic image generation unit that generates a synthetic image in which the ultrasonic echo image and the device position image are combined.

7. An image generation method comprising:
a step of acquiring ultrasonic information of inside of a living body obtained from an ultrasonic probe that has an ultrasonic sensor and emits ultrasonic waves inside the living body;
a step of acquiring device magnetic field information relating to a magnetic field generated from a medical device inserted in a blood vessel inside the living body;
a step of generating an ultrasonic echo image of the blood vessel from the ultrasonic information; and
a step of generating a device position image indicating a position of the medical device inside the blood vessel, based on the ultrasonic echo image and the device magnetic field information.
